(19) Europäisches Patentamt
European Patent Office
Office européen des brevets

(11) EP 1 620 570 B1

(12) EUROPEAN PATENT SPECIFICATION

(45) Date of publication and mention of the grant of the patent:
**15.09.2010 Bulletin 2010/37**

(21) Application number: **04751075.5**

(22) Date of filing: **30.04.2004**

(51) Int Cl.:
***C12Q 1/68*** *(2006.01)*

(86) International application number:
**PCT/US2004/013514**

(87) International publication number:
**WO 2004/099433 (18.11.2004 Gazette 2004/47)**

# (54) ELECTROCHEMICAL METHOD TO MEASURE DNA ATTACHMENT TO AN ELECTRODE SURFACE IN THE PRESENCE OF MOLECULAR OXYGEN

ELEKTROCHEMISCHES VERFAHREN ZUR MESSUNG DER BINDUNG VON DNA AN EINE ELEKTRODENOBERFLÄCHE IN GEGENWART VON MOLEKULAREM SAUERSTOFF

PROCEDE ELECTROMECANIQUE DESTINE A MESURER LA FIXATION DE L'ADN A UNE SURFACE D'ELECTRODE EN PRESENCE D'OXYGENE MOLECULAIRE

(84) Designated Contracting States:
**AT BE BG CH CY CZ DE DK EE ES FI FR GB GR HU IE IT LI LU MC NL PL PT RO SE SI SK TR**

(30) Priority: **02.05.2003 US 429291**

(43) Date of publication of application:
**01.02.2006 Bulletin 2006/05**

(73) Proprietor: **Geneohm Sciences**
**San Diego, CA 92121 (US)**

(72) Inventors:
- **CROTHERS, Donald, M.**
  **Northford, CT 06472 (US)**
- **HOLMLIN, Erik, R.**
  **San Diego, CA 92101 (US)**
- **ZHANG, Honghua**
  **San Diego, CA 92130 (US)**
- **SHI, Chunnian**
  **San Diego, CA 92130 (US)**

(74) Representative: **Raynor, Simon Mark**
**Urquhart-Dykes & Lord LLP**
**Midsummer House**
**413 Midsummer Boulevard**
**Central Milton Keynes MK9 3BN (GB)**

(56) References cited:
**US-A1- 2001 021 534 US-B1- 6 180 346**

- **STEEL ADAM B HERNE TONYA M TARLOV MICHAEL J: "electrochemical quantitation of dna immobilized on gold" 15 November 1998 (1998-11-15), ANALYTICAL CHEMISTRY, AMERICAN CHEMICAL SOCIETY. COLUMBUS, US, PAGE(S) 4670-4677 , XP002960497 ISSN: 0003-2700 * page 4671, column 1, lines 1,2 * * figures 1,3 * * abstract ***
- **STEEL A.B.: 'Electrochemical Quantitation of DNA Immobilized on Gold' ANAL. CHEM. vol. 70, 1998, pages 4670 - 4677, XP002960497**
- **'Gene Characterization Kits' STRATAGENE CATALOG. 1988, page 39, XP002937918**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Related Applications

This application claims priority from U.S. Pat. Application No. 60/424,656 entitled UNIVERSAL TAG ASSAY filed November 6, 2002. This application also claims priority from, and is a continuation-in-part application of U.S. Pat. Application Serial No. 10/424,542 entitled "UNIVERSAL TAG ASSAY," filed April 24, 2003.

### Background of the Invention

### Field of the Invention

This invention relates to a method of detecting nucleic acid hybridization in an electrochemical assay. More particularly, the invention relates to such a method of detecting nucleic acid hybridization in the presence of molecular oxygen. Preferred embodiments include the use of ruthenium amperometry to detect hybridization of DNA or RNA molecules to detection probes immobilized on a detector, preferably a universal chip having gold or carbon electrodes.

### Description of the Related Art

One method to detect nucleic acid hybridization is to detect a quantity of counterions surrounding the nucleic acid. Accordingly, hybridized nucleic acid would tend to be surrounded by more of the counterions than would single stranded nucleic acid. The counterions are typically detected by an electrochemical reaction, for example by reduction of a trivalent ion to divalent; in this way, the counterions function as an electron transfer species.

Electrochemical quantitation is described in A.B. Steel et al., Electrochemical Quantitation of DNA Immobilized on Gold, Anal. Chem. 70:4670-77 (1998). In this publication, Steel *et al.* describe the use of cobalt (III) trisbipyridyl and ruthenium (III) hexaamine as species which interact with surface-immobilized DNA.

The complex $Ru(NH_3)_6^{3+}$ has a reduction potential on a gold electrode of approximately -250 mV versus Ag/AgCl reference. This potential overlaps with the potential range at which diatomic oxygen ($O_2$) is reduced on a gold electrode at neutral pH. If oxygen is present during an assay using $Ru(NH_3)_6^{3+}$, reduction of the oxygen causes a background signal that interferes with the interpretation of the current associated with the reduction of $Ru(NH_3)_6^{3+}$.

One technique used to diminish oxygen's effect is to remove the oxygen in close proximity to the electrochemical cell that would be present in ordinary laboratory conditions. This can be achieved by deaeration with another gas, such as nitrogen or argon. The inert gas is typically administered from a tank to the electrochemical cell before and during the assay to minimize the amount of oxygen present. However, because of the additional steps and equipment involved, deaeration procedures are generally inconvenient, time-consuming, and expensive.

Accordingly, there exists an unmet need in the art for a method of accurately detecting DNA hybridization despite the presence of molecular oxygen in the assay environment.

Adam B. Steel, Tonya M. Heme and Michael J. Tarlov "Electrochemical Quantitation of DNA Immobilized on Gold", Analytical Chemistry 1998, 70, 4670-4677 describe an electrochemical method to quantify the surface density of DNA immobilized on gold. The surface density of DNA is calculated from the amount of cationic redox marker measured at the electrode surface.

Thorp et al, US 6,180,346 describe an electrode and a method of preparing an electrode by electropolymerizing a film on the conductive working surface of an electrode.

### Summary of the Invention

One aspect of the present invention is a method of detecting polynucleotide hybridization, including: providing a probe polynucleotide immobilized to an electrode; contacting the probe with a sample potentially containing target polynucleotide capable of hybridizing with the probe; contacting the probe and any target hybridized thereto with a moiety; and electrochemically determining whether target has hybridized to the probe, wherein no deaeration step is performed prior to electrochemically determining whether target has hybridized to the probe.

One embodiment of the present invention is a methods of detecting polynucleotide hybridization, including: providing a probe polynucleotide immobilized to an electrode; contacting the probe with a sample potentially containing target polynucleotide capable of hybridizing with the probe; contacting the probe and any target hybridized thereto with a ruthenium complex having the formula:

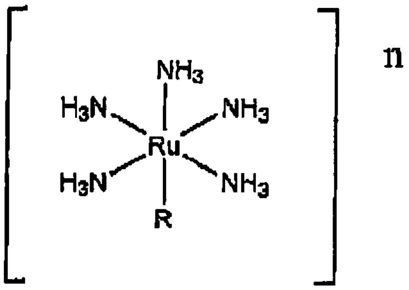

wherein R is an electron withdrawing ligand and n is an integer; and electrochemically determining whether target has hybridized to the probe.

Another embodiment of the invention is a method for detecting a polynucleotide, including: immobilizing a target polynucleotide on an electrode; contacting the target polynucleotide with a ruthenium complex of the formula:

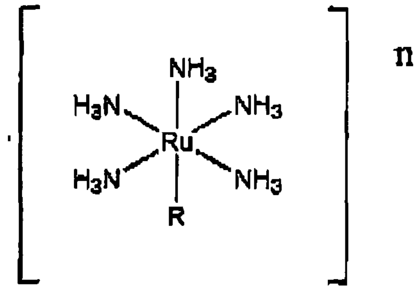

wherein R is an electron withdrawing ligand and n is an integer; and electrochemically detecting the ruthenium complex as an indicator of the presence of immobilized target polynucleotide.

Another embodiment of the invention is a method for quantitating polynucleotide, including: binding polynucleotide to an electrode; contacting the polynucleotide with a ruthenium complex of the formula:

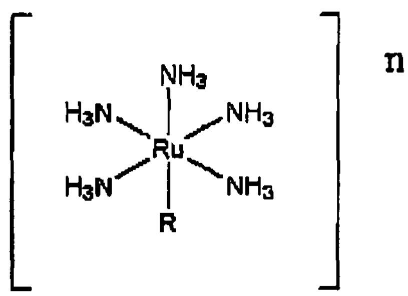

wherein R is an electron withdrawing ligand and n is an integer; and electrochemically detecting the quantity of ruthenium complex associated with the polynucleotide.

Another embodiment of the invention is a method of detecting polynucleotide hybridization, including: providing a probe polynucleotide immobilized to an electrode; providing a target polynucleotide that is substantially longer than the probe polynucleotide and that is potentially capable of hybridizing with the probe polynucleotide; contacting the probe with the target polynucleotide; contacting the probe and any target hybridized thereto with a transition metal complex; and electrochemically determining whether the target has hybridized to the probe.

Another embodiment of the invention is a method of detecting polynucleotide hybridization, including: providing a probe polynucleotide immobilized to an electrode; providing a target polynucleotide that is potentially capable of hybridizing with the probe polynucleotide; contacting the probe with the target polynucleotide; elongating any target polynucleotide that has hybridized to the probe; contacting any hybridized target polynucleotide with a transition metal complex; and electrochemically determining whether the target has hybridized to the probe.

Another embodiment of the invention is a method of detecting polynucleotide hybridization, including: providing a nucleic acid analog probe immobilized to an electrode; providing a target polynucleotide that is potentially capable of hybridizing with the probe; contacting the probe with the target polynucleotide; contacting any hybridized target polynucleotide with a transition metal complex; and electrochemically determining whether the target has hybridized to the probe.

Disclosed a kit for detecting a target polynucleotide, including: an assay device having a binding portion capable of binding target polynucleotide; and a counterion reagent able to associate with the target polynucleotide and instructions for using said assay device for determining electrochemically whether any target polynucleotide has hybridized to said binding portion, in which no deaeration step is performed prior to said determining.

A further embodiment of the invention is a method of detecting polynucleotide hybridization, including: providing a probe polynucleotide immobilized to a non-gold electrode; contacting the probe with a sample potentially containing target polynucleotide capable of hybridizing with the probe; contacting any hybridized target with a moiety having a reduction potential that is substantially different from the reduction potential of diatomic oxygen at the electrode; and electrochemically determining whether target has hybridized to the probe.

## Brief Description of the Drawings

**FIG. 1** shows a voltammogram comparing the reduction potentials of $Ru(NH_3)_5pyridine^{3+}$ and $Ru(NH_3)_6{}^{3+}$.

**FIG. 2** shows a voltammagram which illustrates the signal enhancing effect of on-chip amplification.

## Detailed Description of the Preferred Embodiment

The present invention relates to methods of analyzing a nucleic acid. In preferred embodiments, the nucleic acid comprises DNA. Hence, references to "DNA" are not intended to imply that other nucleic acids or nucleic acid analogs (e.g., RNA, PNA) cannot be used in practicing the present invention, except as so required in the claims.

Ruthenium-based counterions are particularly advantageous in quantitating polynucleotides for the purpose of detecting hybridization. Ruthenium amperometry and the use of the complexes $Ru(NH_3)_6{}^{3+}$ and $Ru(NH_3)_5pyridine^{3+}$ for this purpose are disclosed in copending U.S. Pat. Application No. 60/424656, filed November 6, 2002; U.S. Pat. Application Serial No. 10/424,542 entitled "UNIVERSAL TAG ASSAY," filed April 24, 2003.

It has been discovered that the ligands surrounding the ruthenium atom in various ruthenium complexes determine the reduction potential of the complex. It has further been discovered that if one or more of the six amine groups of ruthenium hexaamine is replaced with an electron withdrawing ligand such as pyridine, the reduction potential of the complex can shift in the positive direction and out of the oxygen reduction window. Use of such a complex greatly improves the measurement of hybridization by this method since the reaction can be performed in the presence of oxygen, eliminating the need for expensive and time-consuming deaeration procedures. Accordingly, some embodiments of the present invention include the use of a counterion having a reduction potential that does not overlap with that of molecular oxygen.

It has also been discovered that the use of electrodes other than gold electrodes can be advantageous for some assays. Electrodes that are not substantially made of gold are herein described as "non-gold" electrodes. Particularly preferred non-gold electrodes include carbon electrodes. For example, when using a carbon electrode instead of a gold electrode, $Ru(NH_3)_6{}^{3+}$ has a reduction potential that does not overlap with the reduction potential of diatomic oxygen. Accordingly, the use of a carbon electrode for an assay in which $Ru(NH_3)_6{}^{3+}$ is a counterion can offset the need to deaerate the assay environment. However, where gold electrodes are preferred to other electrodes, such as carbon electrodes, it is generally advantagous to use a species other than $Ru(NH_3)_6{}^{3+}$ as a counterion.

Ruthenium complexes featuring substituted ligands such as pyridine have previously been studied for other purposes. For example, the attachment of ruthenium pentaamine pyridine complexes to alkanethiols for the study of electron transfer kinetics in self assembled monolayers is described in H.O. Finklea et al., Electron-Transfer Kinetics in Organized Thiol Monolayers with Attached Pentaammine(pyridine)ruthenium Redox Centers, J. Am. Chem. Soc. 114: 3173-3181 (1992) and in H.O. Finklea, Self-assembled Monolayers on Electrodes, Encyclopedia of Analytical Chemistry, John Wiley & Sons Ltd. (2000). The use of $Ru(NH_3)_5py^{3+,2+}$ complexes in electrochemical assays is also described in Hill et al. (U.S. Pat. No. 4,840,893).

Some embodiments of the present invention use a substituted ruthenium pentaamine complex as shown below.

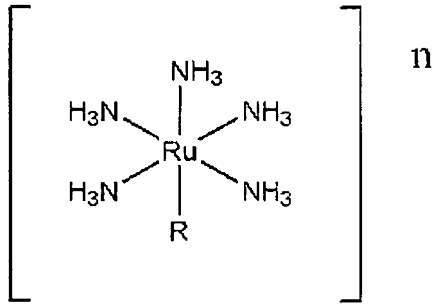

In this structure, R is an electron withdrawing ligand. In some embodiments, the electron withdrawing ligand is a heterocyclic moiety, preferably a nitrogen-containing heterocycle such as substituted or unsubstituted pyridine, pyrimidine, pyridazine, or pyrazine. Other ligands that are suitable for use in the present invention include phosphite derivatives and isonitrile derivatives.

In the structure above, n represents the electrical charge of the complex. Complexes according to the present invention typically carry a positive charge. In some preferred embodiments, the charge is 3+.

Further, some useful counterions include dimers or polymers in which one or more monomeric subunits contain one or more electron withdrawing ligands.

Table 1 depicts several types of ruthenium counterions that are suitable for use with the present invention. For the R group substituents that appear in some of these counterions, Table 1 lists some of the preferred moieties, though it will be appreciated by those of skill in the art that other substituted or unsubstituted alkyl, aryl, and/or heteroatom moieties can also be used.

**Table 1: Structures of Various Ruthenium Complexes**

| Complex | Ligand | Ligand Structure | Substituent |
|---|---|---|---|
| $Ru(NH_3)_5py^{3+}$ | Pyridine derivative | 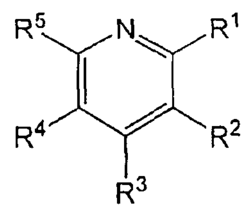 | $R^1$ through $R^5$ = -H, $-CH_3$, $-CO_2CH_3$, -I, -Cl, -Br, $-CH(OH)_2$, $-CH_2OH$, $-CONH_2$, $-CO_2^-$, $-CO_2CH_3$, $-CO_2H$, -CHO |
| $Ru(NH_3)_5pym^{3+}$ | Pyrimidine | ($C_4H_4N_2$, 1.3-Diazine) | |
| $Ru(NH_3)_5Pyd^{3+}$ | Pyridazine | ($C_4H_4N_2$, 1.2-Diazine) | |
| $Ru(NH_3)_5Pz^{3+}$ | Pyrazine | ($C_4H_4N_2$, 1.4-Diazine) | |
| $Ru(NH_3)_5Pi^{3+}$ | Phosphite derivative | 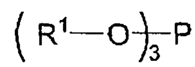 | $R^1$ = -H, $-CH_3$, $-CH_2CH_3$, $-C_6H_5OH$ |
| $Ru(NH_3)_5(CNR)^{3+}$ | Isonitrile derivative | $R^1$-$CH_2$-N=C: | $R^1$ = -H, $-CH_3$, $-CH_2CH_3$, $-C_6H_5OH$ |
| $(NH_3)_5RuPzRu(NH_3)_5^{5+}$ | Pyrazine | ($C_4H_4N_2$, 1.4-Diazine) | |
| $(NH_3)_5RuPzRu(NH_3)_5^{6+}$ | Pyrazine | ($C_4H_4N_2$, 1.4-Diazine) | |
| $[(NH_3)_5RU(PzRu(NH_3)_4)_nPzRu(NH_3)_5]^{+(3n+6)}$ | Rutheniumpentaamine Pyrazine | $PzRu(NH_3)_5^{3+}$ | |
| $[(NH_3)_5Ru(PzRu(NH_3)_4)_nPzRu(NH_3)_5]^{+(3n+4)}$ | Rutheniumpentaamine Pyrazine | $PzRU(NH_3)_5^{2+}$ | |

(continued)

| Complex | Ligand | Ligand Structure | Substituent |
|---|---|---|---|
| $[-(CH_2CBPyRu(NH_3)_5)_n]^{+3n}$ | Polyvinyl pyridine | $-(CH_2CHPy)-$ | |

In some embodiments, counterions can contain more than one electron withdrawing ligand. Such additional ligands can be the same or similar to those indicated above. Further, counterions need not contain ruthenium; for example, other transition metal atoms can be used to create a complex capable of electron transfer. Other chemical moieties that are capable of transferring an electrical charge, preferably by a redox reaction, can also be used as counterions. However, it remains advantageous for a counterion to have a reduction potential that does not overlap with the reduction potential of diatomic oxygen.

The flow of current associated with the reduction of diatomic oxygen on a gold electrode begins to interfere with the assay at approximately -250 mV versus an Ag/AgCl reference. It is preferred that the reduction potential of a counterion used in connection with the present invention be positive of that value. Counterions having a reduction potential that is positive of -200 mV versus an Ag/AgCl reference are preferred. Those that are positive of -100 mV versus an Ag/AgCl reference are more preferred, and those that are approximately 0 mV or positive of 0 mV versus an Ag/AgCl reference are most preferred.

However, it can be advantageous not to use counterions having a reduction potential that is too positive. For example, a counterion that will oxidize water is generally not favorable for use in an aqueous medium. Accordingly, preferred counterions for use in an aqueous assay medium have a reduction potential that is negative of +1.0 V versus an Ag/AgCl reference. More preferably, the reduction potential is negative of +500 mV versus an Ag/AgCl reference. For example, the complex ruthenium(III) pentaamine pyridine has reduction potential that is approximately 0 mV versus an Ag/AgCl reference, and is a particularly preferred counterion.

In some embodiments, the invention uses the metal complex ruthenium(III) pentaamine pyridine ($Ru(NH_3)_5py^{3+}$ shown below), for detecting DNA hybridization at an electrode surface.

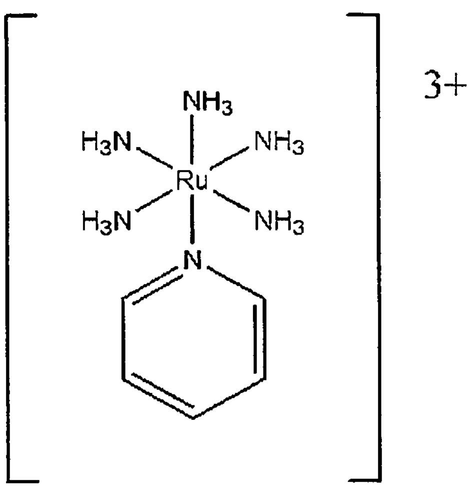

Because of its trivalency, this molecule can associate avidly to negatively charged phosphodiesters of a DNA backbone. In preferred embodiments, the DNA is attached to an electrode to conduct an electrochemical assay. Applying a reducing potential to the electrode causes reduction of the ruthenium. In the case of $Ru(NH_3)_5py^{3+}$, the reduction is typically from the trivalent ion to divalent. This reduction of $Ru(NH_3)_5py^{3+}$ is not convolved with the reduction of oxygen, as is often a problem when using $Ru(NH_3)_6{}^{3+}$.

The quantity of counterions bound to the DNA can be calculated from the number of electrons transferred during the reduction. The following equation can be used to calculate the amount of DNA present based on a measured amount of a redox marker, such as in the electrochemical detection of a ruthenium species:

$$\Gamma_{DNA} = \Gamma_0 (z/m) (N_A)$$

In this equation, $\Gamma_{DNA}$ is the DNA surface density in molecules/$cm^2$, $\Gamma_0$ is the surface density of adsorbed redox marker (mol/$cm^2$), m is the number of bases in the DNA probe, z is the charge of the redox species, and $N_A$ is Avogadro's constant.

FIG. 1 illustrates the separate electrochemical responses for $Ru(NH_3)_5py^{3+}$ and $Ru(NH_3)_6^{3+}$ bound to DNA films on gold electrodes in the presence of oxygen. As shown, the reduction potential of $Ru(NH_3)_5py^{3+}$ is approximately 0 mV, while that of $Ru(NH_3)_6^{3+}$ is approximately -250 to -300 mV.

Accordingly, one advantage of using a species such as $Ru(NH_3)_5py^{3+}$ is that its reduction potential of approximately 0 mV vs. Ag/AgCl is about 300 mV positive of where diatomic oxygen is reduced at a gold electrode. Using $Ru(NH_3)_5py^{3+}$ as a counterion allows a more accurate calculation of the amount of ruthenium (and a more accurate calculation of the amount of nucleic acid) based on the measured current. Measurements of nucleic acid hybridization at an electrode surface may thus be performed in aerated solutions without having to first remove dissolved molecular oxygen.

As indicated above, preferred embodiments of the present invention feature the use of a ruthenium complex in conducting an electrochemical assay. Preferably, such an assay detects nucleic acid hybridization using the general technique of Steele et al. (1998, Anal. Chem 70:4670-4677).

Typically, in carrying out this technique, a plurality of nucleic acid probes which are complementary to a sequence of interest are used. Preferably, these probe strands are immobilized on a surface such as an electrode in contact with a liquid medium. Preferably, the surface is a gold electrode that is coated with a protein layer such as avidin to facilitate the attachment of the nucleic acid probe strands to the electrode. This protein layer should be porous, such that it allows ions to pass from the liquid medium to the electrode and vice versa. Alternatively, probe strands can be attached directly to the surface, for example by using a thiol linkage to covalently bind nucleic acid to a gold electrode.

Next, a target strand (a nucleic acid sample to be interrogated relative to the probe) can be contacted with the probe in any suitable manner known to those skilled in the art. For example, a plurality of target strands can be introduced to the liquid medium and allowed to intermingle with the immobilized probes. Preferably, the number of target strands exceeds the number of probe strands in order to maximize the opportunity of each probe strand to interact with target strands and participate in hybridization. If a target strand is complementary to a probe strand, hybridization can take place. Whether or not hybridization occurs can be influenced by various "stringency" factors such as temperature, pH, or the presence of a species able to denature various hybridized strands. Accordingly, it is often desirable to adjust the assay conditions to achieve a suitable level of stringency; maximum stringency would be a condition in which perfectly complementary strands may hybridize, while all other strands do not. Ideal conditions will generally be those which strike a balance between minimizing the number of hybridizations between noncomplementary strands (false positives) and minimizing the number of probes which remain unhybridized despite the presence of eligible complementary target strands (false negatives). Increasing the quantity and/or size of target strands are examples of techniques that can be useful in minimizing false negatives.

Counterions, such as $Ru(NH_3)_5py^{3+}$, can be introduced to the liquid medium and will tend to cloud around the negatively charged backbones of the various nucleic acid strands. Generally, the counterions will accumulate electrostatically around the phosphate groups of the nucleic acids whether they are single or double stranded. However, because a probe and target together physically constitute a larger amount of DNA than the probe alone, a double stranded DNA will have more counterions surrounding it. Although $Ru(NH_3)_5py^{3+}$ is a preferred counterion, any other suitable transition metal complexes or other counterions that associate with nucleic acid electrostatically and whose reduction or oxidation is electrochemically detectable in an appropriate voltage regime can be used.

Various techniques for measuring the amount of counterions can be used. In a preferred embodiment, amperometry is used to detect an electrochemical reaction at the electrode. Generally, an electrical potential will be applied to the electrode. As the counterions undergo an electrochemical reaction, for example, the reduction of a trivalent ion to divalent at the electrode surface, a measurable current is generated. The amount of current corresponds to the amount of counterions present, which in turn corresponds to the amount of negatively-charged phosphate groups on nucleic acids. Accordingly, measuring the current allows a quantitation of phosphate groups and can allow the operator to distinguish hybridized nucleic acid from unhybridized nucleic acid and determine whether the target being interrogated is complementary to the probe (and contains the sequence of interest).

Alternatively, the measurable distinction between single stranded and double stranded oligonucleotides can be made even more profound. One method is to use target strands which are substantially longer than the probe strands. Accordingly, the longer target strands will accumulate substantially more counterions which will be detectable if the target is hybridized to a probe. A preferred technique for elongating the target strands is rolling circle amplification (RCA). Longer target strands can be made and then introduced to the liquid medium surrounding the probes. Alternatively, it

is possible to increase the length of a target strand after the strand has hybridized to a probe strand. This second technique is often referred to as "on-chip" amplification. Preferred methods of on-chip amplification are head-to-tail polymerization and RCA. On-chip amplification is discussed in greater detail in copending Application Serial No. 10/429,293 entitled "METHOD OF ELECTROCHEMICAL DETECTION OF SOMATIC CELL MUTATIONS," filed May 2, 2003.

Another technique for increasing the signal contrast between single stranded and double stranded DNA is to limit the electrical signal from the probe strands. In particular, this can be done by limiting the electrical attraction between the probe strand and the counterions which participate in electron transfer. For example, if the probe strands are constructed such that they do not contain a negatively charged backbone, then they will not attract counterions. Accordingly, more of the detectable signal will be due to counterions associated with the target strands. In cases where hybridization has not occurred, the detectable signal will be measurably lower since the target strands are not present to participate in counterion attraction.

Probe strands without a negatively charged backbone can include peptide nucleic acids (PNAs), phosphotriesters, methylphosphonates. These nucleic acid analogs are known in the art.

In particular, PNAs are discussed in: Nielsen, "DNA analogues with nonphosphodiester backbones," Annu Rev Biophys Biomol Struct, 1995;24:167-83; Nielsen et al., "An introduction to peptide nucleic acid," Curr Issues Mol Biol, 1999;1(1-2):89-104; and Ray et al., "Peptide nucleic acid (PNA): its medical and biotechnical applications and promise for the future," FASEB J., 2000 Jun;14(9):1041-60;

Phophotriesters are discussed in: Sung *et al.,* "Synthesis of the human insulin gene. Part II. Further improvements in the modified phosphotriester method and the synthesis of seventeen deoxyribooligonucleotide fragments constituting human insulin chains B and mini-CDNA," Nucleic Acids Res, 1979 Dec 20;7(8):2199-212; van Boom et al., "Synthesis of oligonucleotides with sequences identical with or analogous to the 3'-end of 16S ribosomal RNA of Escherichia coli: preparation of m-6-2-A-C-C-U-C-C and A-C-C-U-Cm-4-2C via phosphotriester intermediates," Nucleic Acids Res, 1977 Mar;4(3):747-59; and Marcus-Sekura et al., "Comparative inhibition of chloramphenicol acetyltransferase gene expression by antisense oligonucleotide analogues having alkyl phosphotriester, methylphosphonate and phosphorothioate linkages," Nucleic Acids Res, 1987 Jul 24;15(14):5749-63;

Methylphosphonates are discussed in: U.S. Pat. No. 4,469,863 (Ts'o et al.)*;* Lin et al., "Use of EDTA derivatization to characterize interactions between oligodeoxyribonucleoside methylphophonates and nucleic acids," Biochemistry, 1989, Feb 7;28(3):1054-61; Vyazovkina et al., "Synthesis of specific diastereomers of a DNA methylphosphonate heptamer, d(CpCpApApApCpA), and stability of base pairing with the normal DNA octamer d(TPGPTPTPTPGPGPC)," Nucleic Acids Res, 1994 Jun 25;22(12):2404-9; Le Bec et al., "Stereospecific Grignard-Activated Solid Phase Synthesis of DNA Methylphosphonate Dimers," J Org Chem, 1996 Jan 26;61(2):510-513; Vyazovkina et al., "Synthesis of specific diastereomers of a DNA methylphosphonate heptamer, d(CpCpApApApCpA), and stability of base pairing with the normal DNA octamer d(TPGPTPTPTPGPGPC)," Nucleic Acids Res, 1994 Jun 25;22(12):2404-9; Kibler-Herzog et al., "Duplex stabilities of phosphorothioate, methylphosphonate, and RNA analogs of two DNA 14-mers," Nucleic Acids Res, 1991 Jun 11;19(11):2979-86; Disney et al., "Targeting a Pneumocystis carinii group I intron with methylphosphonate oligonucleotides: backbone charge is not required for binding or reactivity," Biochemistry, 2000 Jun 13;39(23):6991-7000; Ferguson et al., "Application of free-energy decomposition to determine the relative stability of R and S oligodeoxyribonucleotide methylphosphonates," Antisense Res Dev, 1991 Fall; 1(3):243-54; Thiviyanathan et al., "Structure of hybrid backbone methylphosphonate DNA heteroduplexes: effect of R and S stereochemistry," Biochemistry, 2002 Jan 22;41 (3):827-38; Reynolds et al., "Synthesis and thermodynamics of oligonucleotides containing chirally pure R(P) methylphosphonate linkages," Nucleic Acids Res, 1996 Nov 15;24(22):4584-91; Hardwidge et al., "Charge neutralization and DNA bending by the Escherichia coli catabolite activator protein," Nucleic Acids Res, 2002 May 1;30(9):1879-85; and Okonogi et al., "Phosphate backbone neutralization increases duplex DNA flexibility: A model for protein binding," PNAS U.S.A., 2002 Apr 2;99(7):4156-60;

In general, an appropriate nucleic acid analog probe will not contribute, or will contribute less substantially, to the attraction of counterions compared to a probe made of natural DNA. Meanwhile, the target strand will ordinarily feature a natural phosphate backbone having negatively charged groups which attract positive ions and make the strand detectable.

Alternatively, a probe may be constructed that contains both charged nucleic acids and uncharged nucleic acid analogs. Similarly, pure DNA probes can be used alongside probes containing uncharged analogs in an assay. However, precision in distinguishing between single stranded and double stranded will generally increase according to the electrical charge contrast between the probe and the target strands. Hence, the exclusive use of probes made entirely of an uncharged nucleic analog will generally allow the greatest signal contrast between hybridized and non-hybridized molecules on the chip. In general, probe strands containing methylphosphonates are referred. Strands containing phosphotriesters are less preferred since they are generally not soluble in an aqueous medium.

Although techniques (such as those above) for enhancing the measurable distinction between single stranded and double stranded oligonucleotides can be advantageously used in connection with a redox-based assay employing

a counterion capable of reaction at a potential outside the oxygen reduction window, these techniques may also be used in connection with other types of assays and other types of redox species.

Some embodiments of the present invention allow detection of nucleic acid mutations with improved accuracy and precision. In some embodiments, for example, a mutation can be detected at a level of about 1 part in $10^2$ (which means one mutant version of a gene in a sample per 100 total versions of the gene in the sample) or less, about 1 part in $10^3$ or less, about 1 part in $10^4$ or less, about 1 part in $10^5$ or less, or about 1 part in $10^6$ or less.

Disclosed a kit for conducting an assay. Preferably, such a kit includes one or more electrodes, probe sequences which are attached or can be attached to one of the electrodes, and an appropriate counterion reagent. Preferably, the counterion reagent will comprise ruthenium complexes. More preferably, the counterion reagent will comprise $Ru(NH_3)_5py^{3+}$ in a liquid solution. Additionally, a kit according to the present invention can include other reagents and/or devices which are useful in preparing or using any biological samples, electrodes, probe sequences, target sequences, liquid media, counterions, or detection apparatus, for various techniques described herein or already known in the art.

Example 1: Synthesis of $Ru(NH_3)_5pyridine^{3+}$

Ruthenium pentaamine pyridine was synthesized as follows:

1. 0.2 g Chloropentaammineruthenium(III) dichloride ($6.8x10^{-4}$ M) was digested with 4 ml of a solution of silver trifluoroacetate. The resulting solution of chloro-pentaamine ruthenium trifluroacetate was collected after filtration.
2. The ruthenium (III) complex was then reduced by zinc amalgam in the presence of 30-fold excess (about 1 g) of pyridine.
3. After 20 minutes reaction time, saturated ammonium hexafluorophosphate was added to the brilliant yellow reaction mixture to precipitate the yellow solid pentaamminepyridineruthenium (II) hexafluorophosphate, $[(NH_3)_5Ru(py)](PF_6)_3$.
4. The ruthenium complex was then recrystallized from methanol-water mixtures with some loss in yield.
5. A 0.030 g sample of $Ag_2O$ (0.00013 M) was dissolved in 1 ml of water with a minimum of trifluoroacetic acid. In this solution, a portion of 0.00025 M pentaamminepyridineruthenium (II) hexafluorophosphate was digested. The solution was then filtered to remove the resulting metallic silver. Several milliliters of saturated ammonium hexafluorophosphate were then added to yield the solid ruthenium (III) pentaamine pyridine which was collected by filtration and washed with cold ethanol.

Example 2: Performing a Hybridization Assay Using $Ru(NH_3)_5py^{3+}$

A DNA hybridization assay in which $Ru(NH_3)_5py^{3+}$ is used as a counterion can be conducted as follows:

1. A DNA strand is identified as containing a sequence of interest. From this DNA strand, a single stranded oligonucleotide is isolated; this oligonucleotide is approximately 20 bp units in length and contains a sequence that is complementary to the sequence of interest. This oligonucleotide is then amplified by PCR to create probe strands.
2. A gold electrode is provided which contains a porous layer of avidin on its surface. Each probe strand is covalently coupled at one end to a biotin complex. The biotin complexes are then allowed to interact with the avidin on the electrode effectively immobilizing a plurality of probe strands on the electrode surface. Excess probe strands which did not adhere to the avidin layer on the electrode are then washed away using a liquid washing solution.
3. DNA to be interrogated for the sequence of interest is isolated from a tissue sample from a patient. PCR is used to create a plurality of target strands from the region of DNA suspected of containing the sequence of interest.
4. The plurality of target strands is then introduced to a liquid medium in contact with the immobilized probe strands. The quantity of target strands substantially exceeds the quantity of immobilized probe strands. The temperature, pH, and contents of the liquid medium are adjusted so as to allow a target strand to hybridize to an immobilized probe only if the target strand contains a sequence that is perfectly complementary to that of the probe.
5. After the target strands have interacted with immobilized probes, excess unbound target strands are washed away using a liquid washing solution.
6. A liquid solution containing $Ru(NH_3)_5py^{3+}$ ions is introduced to the electrode surface containing the immobilized nucleic acid strands.
7. An electrical potential is applied to the electrode. Current, corresponding to the reduction of trivalent ruthenium complexes to divalent, is measured at the electrode surface.
8. The amount of measured current is evaluated to determine whether it likely corresponds to single stranded or double stranded nucleic acid. This determination is used to conclude whether the DNA being interrogated contains the sequence of interest.

Example 3: Augmenting an Electrical Signal Using On-chip Amplification

The following procedure was performed to determine the effectiveness of on-chip amplification for enhancing an electrical signal. The results are discussed with reference to FIG. 2

To prepare a nucleic acid film on the surface of an electrode, 1.5 μl of a solution consisting of biotinylated capture probe and NeutrAvidin was deposited to the surface of a carbon electrode and allowed to air dry.

The carbon electrode was transferred to a solution containing 5 μM of $Ru(NH_3)_6Cl_3$ in 10 mM Tris + 10 mM NaCl. The electrochemical response was detected and recorded using Osteryoung Square Wave Voltammetry (OSWV). This measurement corresponds to the quantity of nucleic acid immobilized on the electrode before RCA is performed. The resulting current is represented by the smaller curve (which peaks at approximately 0.100 μA) as depicted in FIG. 2.

The immobilized capture probe was then hybridized with a circularized DNA as follows. The electrode was rinsed with Tris buffer solution. Then, 10 μl of solution containing circularized DNA in 10 mM Hepes + 1 M LiCl was applied to the surface of the carbon electrode. The electrode was maintained at 60 ˚C for 5 minutes and then cooled to room temperature and maintained at the room temperature for 30 minutes.

RCA was then performed as follows. The electrode was rinsed with the Tris buffer. A mixture of RCA working solution containing phi29 polymerase and dNTPs in tris buffer was applied. RCA was allowed to proceed at 37 ˚C for 1 hour.

The electrode was rinsed with tris buffer and transferred to a solution containing 5 μM of $Ru(NH_3)_6Cl_3$ in 10 mM Tris + 10 mM NaCl. The electrochemical response was again detected and recorded using OSWV. The resulting current is represented by the larger curve (which peaks at approximately 1.800 μA) as depicted in FIG. 2.

The change from the smaller curve to the larger curve corresponds to the increased number of ruthenium complexes which associate with the more numerous $PO_3^-$ moieties on a larger amount of DNA.

## Claims

**1.** A method of detecting polynucleotide hybridization, comprising:

providing a probe polynucleotide immobilized to an electrode;
contacting the probe with a sample potentially containing target polynucleotide capable of hybridizing with the probe;
contacting the probe and any target hybridized thereto with a ruthenium complex comprising an electron withdrawing ligand, said ruthenium complex having a reduction potential that is positive relative to and does not overlap with the reduction potential of diatomic oxygen; and
electrochemically determining whether target has hybridized to the probe, wherein no deaeration step is performed prior to electrochemically determining whether target has hybridized to the probe.

**2.** The method of Claim 1 wherein the electron withdrawing ligand is selected from the group consisting of pyridine, a pyridine derivative, pyrimidine, pyridazine, pyrazine, a phosphite derivative, an isonitrile derivative, rutheniumpentaamine pyrazine, and polyvinyl pyridine.

**3.** The method of Claim 1 wherein the ruthenium complex has a reduction potential at the electrode that is positive of -200 mV versus an Ag/AgCl reference.

**4.** The method of Claim I wherein the ruthenium complex has a reduction potential at the electrode that is positive of -100 mV versus an Ag/AgCl reference.

**5.** The method of Claim 1 wherein the ruthenium complex has a reduction potential at the electrode that is positive of 0 mV versus an Ag/AgCl reference.

**6.** The method of Claim 1 wherein the ruthenium complex has a reduction potential at the electrode that is between -100 mV and +200 mV versus an Ag/AgCl reference.

**7.** The method or Claim 1 wherein the probe polynucleotide comprises DNA.

**8.** The method of Claim 1 wherein the probe polynucleotide comprises PNA.

**9.** The method of Claim 1 wherein the probe polynucleotide comprises methylphosphonate.

**10.** The method of Claim 1 wherein the probe polynucleotide comprises phosphotriester.

**11.** The method of Claim 1 wherein the probe polynucleotide comprises RNA.

**12.** The method of Claim 1 wherein the target polynucleotide comprises DNA.

**13.** The method of Claim 1 wherein the target polynucleotide comprises RNA.

**14.** The method of Claim 1, wherein said ruthenium complex has the formula:

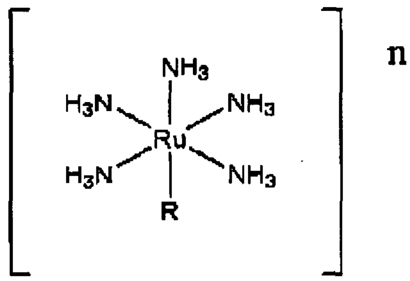

wherein R is an electron withdrawing ligand and n is an integer.

**15.** The method of Claim 14 wherein R is pyridine or a pyridine derivative and n is 3+ or 2+.

**16.** The method of Claim 14 wherein the ruthenium complex is ruthenium (III) pentaamine pyridine.

**17.** The method of Claim 14 wherein determining electrochemically whether said target has hybridized to said probe comprises amperometry.

**18.** The method of Claim 14 wherein the step of determining electrochemically whether said target has hybridized to said probe is performed in the presence of molecular oxygen in a concentration that is substantially equal to ambient conditions.

**19.** The method of Claim 1 wherein said ruthenium complex has the formula:

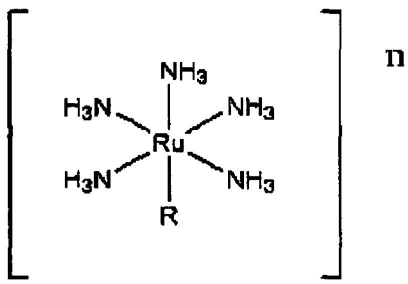

wherein R is an electron withdrawing ligand and n is an integer; and
wherein said electrochemically determining whether target has hybridized to the probe comprises electrochemically detecting the ruthenium complex as an indicator of the presence of immobilized target polynucleotide.

**20.** The method of Claim 19, wherein detecting comprises quantitating the target polynucleotide.

**21.** The method of Claim 19, wherein the detecting step is performed in the presence of molecular oxygen.

**22.** The method of Claim 1, wherein said ruthenium complex has the formula:

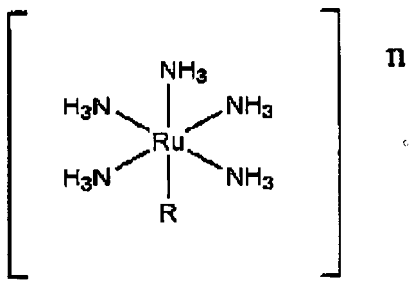

wherein R is an electron withdrawing ligand and n is an integer; and wherein said electrochemically determining whether target has hybridized to the probe comprises electrochemically detecting the quantity of ruthenium complex associated with the polynucleotide as an indicator of the quantity of polynucleotide present..

**23.** The method of Claim 22, wherein the detecting step is performed in the presence of molecular oxygen.

**24.** The method of Claim 1 wherein said target polynucleotide is substantially longer than said probe polynucleotide.

**25.** The method of Claim 24 wherein said ruthenium complex is ruthenium (III) pentaamine pyridine.

**26.** The method of Claim 1, further comprising:

elongating any target polynucleotide that has hybridized to said probe.

**27.** The method of Claim 26 wherein said elongating comprises head-to-tail polymerization.

**28.** The method of Claim 26 wherein said elongating comprises RCA.

**29.** The method of Claim 26 wherein said ruthenium complex is ruthenium (III) pentaamine pyridine.

**30.** The method of Claim 1, wherein said probe polynucleotide comprises a nucleic acid analog.

**31.** The method of Claim 30 wherein said nucleic acid analog probe comprises PNA.

**32.** The method of Claim 30 wherein said nucleic acid analog probe comprises methylphosphonate.

**33.** The method of Claim 30 wherein said nucleic acid analog probe comprises phosphotriester.

**34.** The method of Claim 30 wherein said ruthenium complex is ruthenium (III) pentaamine pyridine.

**35.** A method of detecting polynucleotide hybridization, comprising:

providing a probe polynucleotide immobilized to a carbon electrode;
contacting the probe with a sample potentially containing target polynucleotide capable of hybridizing with the probe;
contacting the probe and any target hybridized thereto with a ruthenium complex comprising $Ru(NH_3)_6^{3+}$, said ruthenium complex having a reduction potential that does not overlap with the reduction potential of diatomic oxygen; and
electrochemically determining whether target has hybridized to the probe, wherein no deaeration step is performed prior to electrochemically determining whether target has hybridized to the probe.

## Patentansprüche

**1.** Verfahren zum Nachweisen von Polynukleotidhybridisierung, umfassend:

Bereitstellen eines Sonden-Polynukleotids, das an einer Elektrode immobilisiert ist;

Kontaktieren der Sonde mit einer Probe, die möglicherweise Target-Polynukleotid enthält, welches mit der Sonde hybridisieren kann;
Kontaktieren der Sonde und etwaig daran hybridisierten Targets mit einem Rutheniumkomplex, welcher einen elektronenziehenden Liganden umfasst, wobei der Rutheniumkomplex ein Reduktionspotential hat, welches positiv gegenüber dem Reduktionspotential von zweiatomigem Sauerstoff ist und nicht mit demselben überlappt; und
elektrochemisches Bestimmen, ob Target mit der Sonde hybridisiert hat, wobei vor dem elektrochemischen Bestimmen, ob Target mit der Sonde hybridisiert hat, kein Entlüftungsschritt ausgeführt wird.

**2.** Verfahren nach Anspruch 1, wobei der elektronenziehende Ligand ausgewählt ist aus der Gruppe bestehend aus: Pyridin, ein Pyridinderivat. Pyrimidin, Pyridazin, Pyrazin, ein Phosphitderivat, ein Isonitrilderivat, Rutheniumpentaaminpyrazin und Polyvinylpyridin.

**3.** Verfahren nach Anspruch 1, wobei der Rutheniumkomplex ein Reduktionspotential an der Elektrode hat, welches positiver als -200 mV gegenüber einer Ag/AgCl-Referenz ist.

**4.** Verfahren nach Anspruch 1, wobei der Rutheniumkomplex ein Reduktionspotential an der Elektrode hat, welches positiver als -100 mV gegenüber einer Ag/AgCl-Referenz ist.

**5.** Verfahren nach Anspruch 1, wobei der Rutheniumkomplex ein Reduktionspotential an der Elektrode hat, welches positiver als 0 mV gegenüber einer Ag/AgCl-Referenz ist.

**6.** Verfahren nach Anspruch 1, wobei der Rutheniumkomplex ein Reduktionspotential an der Elektrode hat, welches zwischen -100 mV und +200 mV gegenüber einer Ag/AgCl-Referenz ist.

**7.** Verfahren nach Anspruch 1, wobei das Sonden-Polynukleotid DNA umfasst.

**8.** Verfahren nach Anspruch 1, wobei das Sonden-Polynukleotid PNA umfasst.

**9.** Verfahren nach Anspruch 1, wobei das Sonden-Polynukleotid Methylphosphonat umfasst.

**10.** Verfahren nach Anspruch 1, wobei das Sonden-Polynukleotid Phosphotriester umfasst.

**11.** Verfahren nach Anspruch 1, wobei das Sonden-Polynukleotid RNA umfasst.

**12.** Verfahren nach Anspruch 1, wobei das Target-Polynukleotid DNA umfasst.

**13.** Verfahren nach Anspruch 1, wobei das Target-Polynukleotid RNA umfasst.

**14.** Verfahren nach Anspruch 1, wobei der Rutheniumkomplex die Formel:

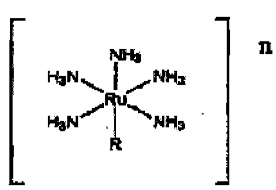

hat, wobei R ein elektronenziehender Ligand ist und n eine ganze Zahl ist.

**15.** Verfahren nach Anspruch 14, wobei R Pyridin oder ein Pyridinderivat ist und n 3+ oder 2+ ist.

**16.** Verfahren nach Anspruch 14, wobei der Rutheniumkomplex Ruthenium(III)pentaaminpyridin ist.

**17.** Verfahren nach Anspruch 14, wobei das elektrochemische Bestimmen, ob das Target mit der Sonde hybridisiert hat. Amperometrie umfasst.

**18.** Verfahren nach Anspruch 14, wobei der Schritt des elektrochemischen Bestimmens, ob das Target mit der Sonde hybridisiert hat, in Gegenwart von molekularem Sauerstoff in einer Konzentration ausgeführt wird, die im wesentlichen gleich den Umgebungsbedingungen ist.

**19.** Verfahren nach Anspruch 1, wobei der Rutheniumkomplex die Formel:

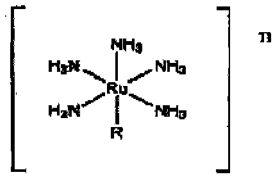

hat, wobei
R ein elektronenziehender Ligand ist und n eine ganze Zahl ist; und
wobei das elektrochemische Bestimmen, ob Target mit der Sonde hybridisiert hat, das elektrochemische Nachweisen des Rutheniumkomplexes als Indikator für das Vorhandensein immobilisierten Target-Polynukleotids umfasst.

**20.** Verfahren nach Anspruch 19, wobei das Nachweisen das Quantifizieren des Target-Polynukleotids umfasst.

**21.** Verfahren nach Anspruch 19, wobei der Nachweisschritt in Gegenwart von molekularem Sauerstoff ausgeführt wird.

**22.** Verfahren nach Anspruch 1, wobei der Rutheniumkomplex die Formel:

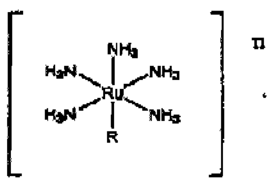

hat, wobei
R ein elektronenziehender Ligand ist und n eine ganze Zahl ist; und
wobei das elektrochemische Bestimmen, ob Target mit der Sonde hybridisiert hat, das elektrochemische Bestimmen der Menge des Rutheniumkomplexes, der mit dem Polynukleotid verbunden ist, als Indikator für die Menge an vorhandenem Polynukleotid umfasst.

**23.** Verfahren nach Anspruch 22, wobei der Nachweisschritt in Gegenwart von molekularem Sauerstoff ausgeführt wird.

**24.** Verfahren nach Anspruch 1, wobei das Zielpolynukleotid wesentlich länger als das Sonden-Polynukleotid ist.

**25.** Verfahren nach Anspruch 24, wobei der Rutheniumkomplex Ruthenium(III)pentaaminpyridin ist.

**26.** Verfahren nach Anspruch 1, ferner umfassend:

Verlängern etwaigen Zielpolynukleotids, das mit der Sonde hybridisiert hat.

**27.** Verfahren nach Anspruch 26, wobei das Verlängern eine Kopf-Schwanz-Polymerisation umfasst.

**28.** Verfahren nach Anspruch 26, wobei das Verlängern RCA umfasst.

**29.** Verfahren nach Anspruch 26, wobei der Rutheniumkomplex Ruthenium(III)pentaaminpyridin ist.

**30.** Verfahren nach Anspruch 1, wobei das Sonden-Polynukleotid ein Nukleinsäure-Analogon umfasst.

**31.** Verfahren nach Anspruch 30, wobei die Nukleinsäure-Analogon-Sonde PNA umfasst.

**32.** Verfahren nach Anspruch 30, wobei die Nukleinsäure-Analogon-Sonde Methylphosphonat umfasst.

**33.** Verfahren nach Anspruch 30, wobei die Nukleinsäure-Analogon-Sonde Phosphotriester umfasst.

**34.** Verfahren nach Anspruch 30. wobei der Rutheniumkomplex Ruthenium(III)pentaaminpyridin ist.

**35.** Verfahren zum Nachweisen von Polynukleotidhybridisierung, umfassend:

Bereitstellen eines Sonden-Polynukleotids, das an einer Kohlenstoffelektrode immobilisiert ist;
Kontaktieren der Sonde mit einer Probe, die möglicherweise Target-Polynukleotid enthält, welches mit der Sonde hybridisieren kann;
Kontaktieren der Sonde und etwaig daran hybridisierten Targets mit einem Rutheniumkomplex, welcher $Ru(NH_3)_6^{3+}$ umfasst, wobei der Rutheniumkomplex ein Reduktionspotential hat, welches nicht mit dem Reduktionspotential von zweiatomigem Sauerstoff überlappt; und
elektrochemisches Bestimmen, ob Target mit der Sonde hybridisiert hat, wobei vor dem elektrochemischen Bestimmen, ob Target mit der Sonde hybridisiert hat, kein Entlüftungsschritt ausgeführt wird.

**Revendications**

**1.** Procédé pour détecter une hybridation de polynucléotides, comprenant les étapes consistant à :

mettre en oeuvre une sonde polynucléotidique immobilisée sur une électrode ;
mettre en contact la sonde avec un échantillon contenant potentiellement un polynucléotide cible capable de s'hybrider à la sonde ;
mettre en contact la sonde et toute cible hybridée à celle-ci avec un complexe de ruthénium comprenant un ligand électroattracteur, ledit complexe de ruthénium ayant un potentiel de réduction positif par rapport au potentiel de réduction de l'oxygène diatomique et ne le chevauche pas; et
déterminer par voie électrochimique si la cible s'est hybridée à la sonde, dans lequel on ne procède pas à une étape de désaération avant de déterminer par voie électrochimique si la cible s'est hybridée à la sonde.

**2.** Procédé selon la revendication 1, dans lequel le ligand électroattracteur est choisi dans le groupe comprenant la pyridine, un dérivé de pyridine, la pyrimidine, la pyridazine, la pyrazine, un dérivé du phosphite, un dérivé d'isonitrile, la ruthénium-pentaamine-pyrazine et la polyvinylpyridine.

**3.** Procédé selon la revendication 1, dans lequel le complexe de ruthénium présente à l'électrode un potentiel de réduction qui est de -200 mV positif par rapport à une référence d'Ag/AgCl.

**4.** Procédé selon la revendication 1, dans lequel le complexe de ruthénium présente à l'électrode un potentiel de réduction qui est de -100 mV positif par rapport à une référence d'Ag/AgCl.

**5.** Procédé selon la revendication 1, dans lequel le complexe de ruthénium présente à l'électrode un potentiel de réduction qui est de 0 mV positif par rapport à une référence d'Ag/AgCl.

**6.** Procédé selon la revendication 1, dans lequel le complexe de ruthénium présente à l'électrode un potentiel de réduction qui est de -100 mV positif par rapport à une référence d'Ag/AgCl.

**7.** Procédé selon la revendication 1, dans lequel la sonde polynucléotidique comprend de l'ADN.

**8.** Procédé selon la revendication 1, dans lequel la sonde polynucléotidique comprend des plynucléaires aromatiques.

**9.** Procédé selon la revendication 1, dans lequel la sonde polynucléotidique comprend du méthylphosphonate.

**10.** Procédé selon la revendication 1, dans lequel la sonde polynucléotidique comprend du phosphotriester.

**11.** Procédé selon la revendication 1, dans lequel la sonde polynucléotidique comprend de l'ARN.

**12.** Procédé selon la revendication 1, dans lequel le polynucléotide cible comprend de l'ADN.

13. 8.Procédé selon la revendication 1, dans lequel le polynucléotide cible comprend de l'ARN.

14. Procédé selon la revendication 1, dans lequel ledit complexe de ruthénium répond à la formule suivante :

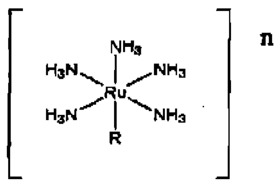

dans laquelle R représente un ligand électroattracteur et n est un nombre entier.

15. Procédé selon la revendication 14, dans lequel R représente la pyridine ou un dérivé de pyridine et n vaut 3+ ou 2+.

16. Procédé selon la revendication 14, dans lequel le complexe de ruthénium est la ruthénium(III)-pentaamine-pyridine.

17. Procédé selon la revendication 14, dans lequel déterminer par voie électrochimique si ladite cible s'est hybridée à ladite sonde comprend l'ampérométrie.

18. Procédé selon la revendication 14, dans lequel l'étape consistant à déterminer par voie électrochimique si ladite cible s'est hybridée à ladite sonde est exécutée en présence d'oxygène moléculaire en concentration sensiblement égale à celle des conditions ambiantes.

19. Procédé selon la revendication 1, dans lequel ledit complexe de ruthénium répond à la formule suivante :

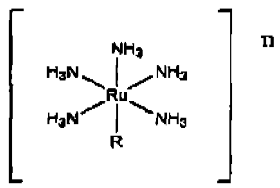

dans laquelle R représente un ligand électroattracteur et n est un nombre entier ; et
dans lequel ladite détermination électrochimique permettant de savoir si la cible s'est hybridée à la sonde comprend la détection électrochimique du complexe de ruthénium comme indicateur de la présence d'un polynucléotide cible immobilisé.

20. Procédé selon la revendication 19, dans lequel la détection comprend la quantification du polynucléotide cible.

21. Procédé selon la revendication 19, dans lequel l'étape de détection est exécutée en présence d'oxygène moléculaire.

22. Procédé selon la revendication 1, dans lequel ledit complexe de ruthénium répond à la formule suivante :

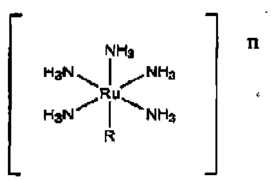

dans laquelle R représente un ligand électroattracteur et n est un nombre entier ; et dans lequel ladite détermination électrochimique permettant de savoir si une cible s'est hybridée à la sonde comprend la détection électrochimique de la quantité de complexe de ruthénium associée au polynucléotide comme indicateur de la quantité de polynucléotide présente.

23. Procédé selon la revendication 22, dans lequel l'étape de détection est exécutée en présence d'oxygène moléculaire.

24. Procédé selon la revendication 1, dans lequel ledit polynucléotide cible est sensiblement plus long que ladite sonde polynucléotidique.

25. Procédé selon la revendication 24, dans lequel ledit complexe de ruthénium est la ruthénium(III)-pentaamine-pyridine.

26. Procédé selon la revendication 1, comprenant en outre l'étape consistant à élonguer tout polynucléotide cible qui s'est hybridé à ladite sonde.

27. Procédé selon la revendication 26, dans lequel ladite élongation comprend une polymérisation de tête à queue.

28. Procédé selon la revendication 26, dans lequel ladite élongation comprend la réplication circulaire (RCA).

29. Procédé selon la revendication 26, dans lequel ledit complexe de ruthénium est la ruthénium(III)-pentaamine-pyridine.

30. Procédé selon la revendication 1, dans lequel ladite sonde polynucléotidique comprend un analogue d'acide nucléique.

31. Procédé selon la revendication 30, dans lequel ladite sonde à base d'analogue d'acide nucléique comprend des polynucléotides aromatiques.

32. Procédé selon la revendication 30, dans lequel ladite sonde à base d'analogue d'acide nucléique comprend un méthylphosphonate.

33. Procédé selon la revendication 30, dans lequel ladite sonde à base d'analogue d'acide nucléique comprend un phosphotriester.

34. Procédé selon la revendication 30, dans lequel ledit complexe de ruthénium est la ruthénium(III)-pentaamine-pyridine.

35. Procédé pour détecter une hybridation de polynucléotides, comprenant les étapes consistant à : mettre en oeuvre une sonde polynucléotidique immobilisée sur une électrode de carbone ;
mettre en contact la sonde avec un échantillon contenant potentiellement un polynucléotide cible capable de s'hybrider à la sonde ;
mettre en contact la sonde avec toute cible hybridée à cette dernière avec un complexe de ruthénium comprenant du $Ru(NH_3)_6^{3+}$, ledit complexe de ruthénium ayant un potentiel de réduction qui ne chevauche pas le potentiel de réduction de l'oxygène diatomique ; et
déterminer par voie électrochimique si la cible s'est hybridée à la sonde, où l'on ne procède à aucune étape de désaération avant de déterminer par voie électrochimique si la cible s'est hybridée à la sonde.

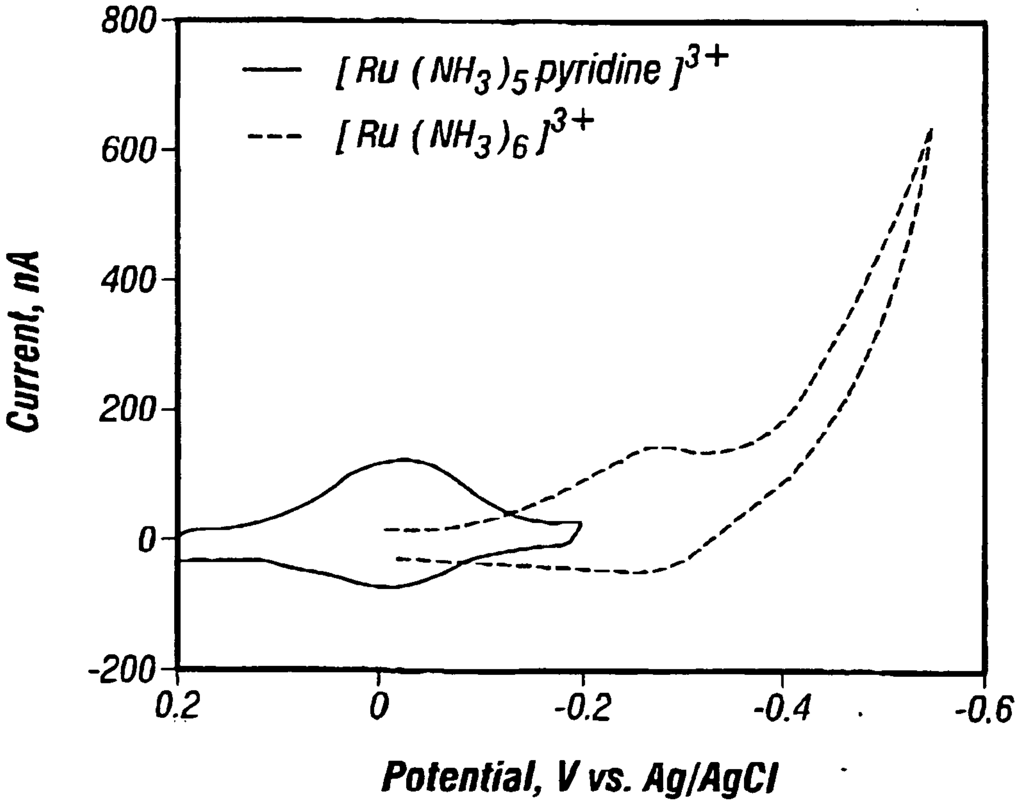
800
600
400
200
0
-200
Current, nA
[Ru (NH3)5 pyridine ]3+
[Ru (NH3)6 ]3+
0.2
0
-0.2
-0.4
-0.6
Potential, V vs. Ag/AgCl

FIG. 1

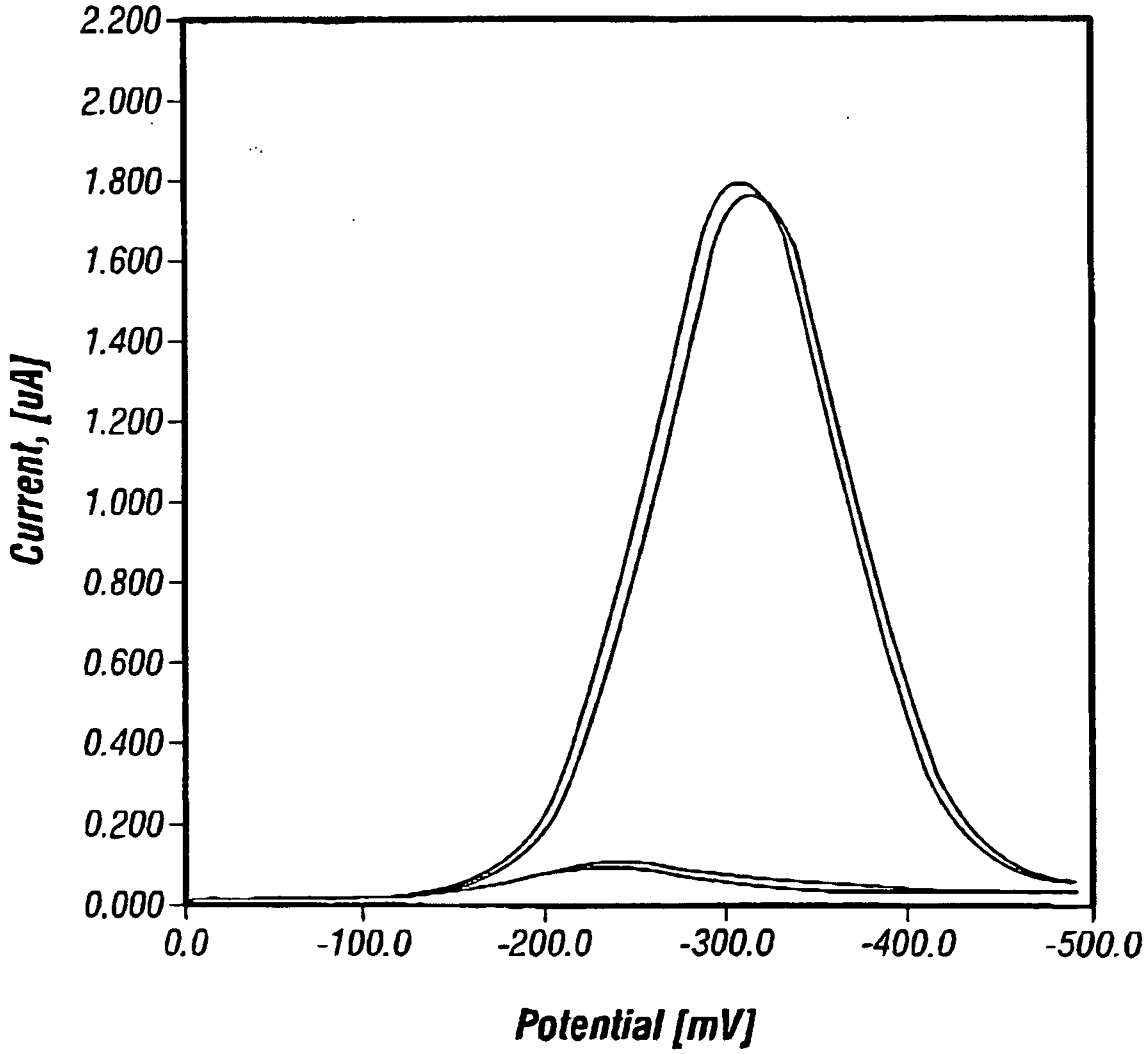
2.200
2.000
1.800
1.600
1.400
1.200
1.000
0.800
0.600
0.400
0.200
0.000
Current, [uA]
0.0
-100.0
-200.0
-300.0
-400.0
-500.0
Potential [mV]

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 42465602 P
- US 42454203 A
- US 6180346 B, Thorp
- US 4840893 A, Hill
- US 10429293 B
- US 4469863 A, Ts'o


### Non-patent literature cited in the description


- **A.B. Steel et al.** Electrochemical Quantitation of DNA Immobilized on Gold. *Anal. Chem.,* 1998, vol. 70, 4670-77
- **Adam B. Steel ; Tonya M. Heme ; Michael J. Tarlov.** Electrochemical Quantitation of DNA Immobilized on Gold. *Analytical Chemistry,* 1998, vol. 70, 4670-4677
- **H.O. Finklea et al.** Electron-Transfer Kinetics in Organized Thiol Monolayers with Attached Pentaammine(pyridine)ruthenium Redox Centers. *J. Am. Chem. Soc.,* 1992, vol. 114, 3173-3181
- Self-assembled Monolayers on Electrodes. **H.O. Finklea.** Encyclopedia of Analytical Chemistry. John Wiley & Sons Ltd, 2000
- **Steele et al.** *Anal. Chem,* 1998, vol. 70, 4670-4677
- **Nielsen.** DNA analogues with nonphosphodiester backbones. *Annu Rev Biophys Biomol Struct,* 1995, vol. 24, 167-83
- **Nielsen et al.** An introduction to peptide nucleic acid. *Curr Issues Mol Biol,* 1991, vol. 1 (1-2), 89-104
- **Ray et al.** Peptide nucleic acid (PNA): its medical and biotechnical applications and promise for the future. *FASEB J.,* June 2000, vol. 14 (9), 1041-60
- *Nucleic Acids Res,* 20 December 1979, vol. 7 (8), 2199-212
- **van Boom et al.** Synthesis of oligonucleotides with sequences identical with or analogous to the 3'-end of 16S ribosomal RNA of Escherichia coli: preparation of m-6-2-A-C-C-U-C-C and A-C-C-U-Cm-4-2C via phosphotriester intermediates. *Nucleic Acids Res,* March 1977, vol. 4 (3), 747-59
- **Marcus-Sekura et al.** Comparative inhibition of chloramphenicol acetyltransferase gene expression by antisense oligonucleotide analogues having alkyl phosphotriester, methylphosphonate and phosphorothioate linkages. *Nucleic Acids Res,* 24 July 1987, vol. 15 (14), 5749-63
- **Lin et al.** Use of EDTA derivatization to characterize interactions between oligodeoxyribonucleoside methylphophonates and nucleic acids. *Biochemistry,* 07 February 1989, vol. 28 (3), 1054-61
- **Vyazovkina et al.** Synthesis of specific diastereomers of a DNA methylphosphonate heptamer, d(CpCpApApApCpA), and stability of base pairing with the normal DNA octamer d(TPGPTPTPTPGPGPC). *Nucleic Acids Res,* 25 June 1994, vol. 22 (12), 2404-9
- **Le Bec et al.** Stereospecific Grignard-Activated Solid Phase Synthesis of DNA Methylphosphonate Dimers. *J Org Chem,* 26 January 1996, vol. 61 (2), 510-513
- **Kibler-Herzog et al.** Duplex stabilities of phosphorothioate, methylphosphonate, and RNA analogs of two DNA 14-mers. *Nucleic Acids Res,* 11 June 1991, vol. 19 (11), 2979-86
- **Disney et al.** Targeting a Pneumocystis carinii group I intron with methylphosphonate oligonucleotides: backbone charge is not required for binding or reactivity. *Biochemistry,* 13 June 2000, vol. 39 (23), 6991-7000
- **Ferguson et al.** Application of free-energy decomposition to determine the relative stability of R and S oligodeoxyribonucleotide methylphosphonates. *Antisense Res Dev,* 1991, vol. 1 (3), 243-54
- **Thiviyanathan et al.** Structure of hybrid backbone methylphosphonate DNA heteroduplexes: effect of R and S stereochemistry. *Biochemistry,* 22 January 2002, vol. 41 (3), 827-38
- **Reynolds et al.** Synthesis and thermodynamics of oligonucleotides containing chirally pure R(P) methylphosphonate linkages. *Nucleic Acids Res,* 15 November 1996, vol. 24 (22), 4584-91
- **Hardwidge et al.** Charge neutralization and DNA bending by the Escherichia coli catabolite activator protein. *Nucleic Acids Res,* 01 May 2002, vol. 30 (9), 1879-85
- **Okonogi et al.** Phosphate backbone neutralization increases duplex DNA flexibility: A model for protein binding. *PNAS U.S.A.,* 02 April 2002, vol. 99 (7), 4156-60